# EUROPEAN PATENT SPECIFICATION

(11) **EP 1 472 987 B1**
(45) Date of publication and mention of the grant of the patent: **09.01.2008**
(21) Application number: 04101763.3
(22) Date of filing: 27.04.2004
(51) Int. Cl.: A61B 19/02, B65D 77/04

(54) **A container for collecting and transporting special waste**
Behälter zum Sammeln und Transportieren von Sondermüll
Réceptacle collecteur et de transport de déchets spéciaux

(30) Priority: 02.05.2003 NL 1023328
(43) Date of publication of application: 03.11.2004
(73) Proprietor: Ecama Holding B.V., 3862 WL Nijkerk (NL)
(72) Inventor: Versluis, Evert, Cornelis, Antonie, 3862 WL, Nijkerk (NL)
(74) Representative: de Vries, Johannes Hendrik Fokke

(56) References cited:
- EP-A- 0 351 483
- WO-A-93/21851
- GB-A- 1 364 053
- US-A- 4 863 052
- US-A- 5 687 839

## Description

The invention relates to a container for collecting and transporting special waste, in particular hospital waste, comprising a closable outer box made of cardboard, an insert made of cardboard, which can be placed in the outer box, and a liner of plastic material, which is positioned between the outer box and the inner box.

Such a container may e.g. be used in a method for collecting and transporting waste according to EP-A-1 153 855. Like US-A-4,978,028 and EP-A-0 351 483, said publication describes a container of the above kind. With the known containers, the liner of plastic material that is positioned between the outer box and the inner box consists of a plastic bag, which loosely surrounds the inner box and which is sealed by means of a sealing clip, a labyrinth seal or a sliding seal after the inner box has been filled. Such sealing means have the drawback that it is not possible in practice to obtain an adequate, leak-proof seal of the plastic bag. In addition, excess material of the plastic bag is present between the inner box and the outer box at the upper side of the container, as a result of which the space in the outer box is not optimally utilised for waste.

With the container according to US-A-4 978 028, the outer box and the insert are made of corrugated cardboard, and a loose inner box bottom and a special cover are used. The corrugated cardboard that is used is not waterproof, however, so that there is a considerable risk of the container being pierced. Spacers positioned between the insert and the outer box provide an interspace between the insert and the outer box.

A container according to the preamble of claim 1 is known from document GB-A-1 364 053.

The object of the invention as defined in claim 1 is to provide an improved container of the above kind.

In this way a container is obtained in which a simple, effective, leak-proof closure of the filling opening is possible through the use of a foil that is provided around the inner box with a close fit, on the end flaps of which an adhesive is present. The container according to the invention guarantees a safe storage of the special waste under all circumstances, also if the waste comprises a liquid, with the inner box remaining resistant to piercing. This enables safe transportation of the special waste, in particular when using the aforesaid method according to EP-A-1 153 855.

The invention furthermore provides a method as defined in claim 11. for manufacturing a container according to the invention.

The invention will now be explained in more detail with reference to the drawing, which very schematically shows an embodiment of the system according to the invention.
Fig. 1 schematically shows an embodiment of the container according to the invention, with the inner box positioned outside the outer box.
Fig. 2 is a perspective view of the inner box of the container of Fig. 1 prior to the provision of the liner of plastic material.
Fig. 3 is a top plan view of the blank for forming the inner box of the container of Fig. 1.
Fig. 4 is a top plan view of the blank for forming the outer box of the container of Fig. 1.

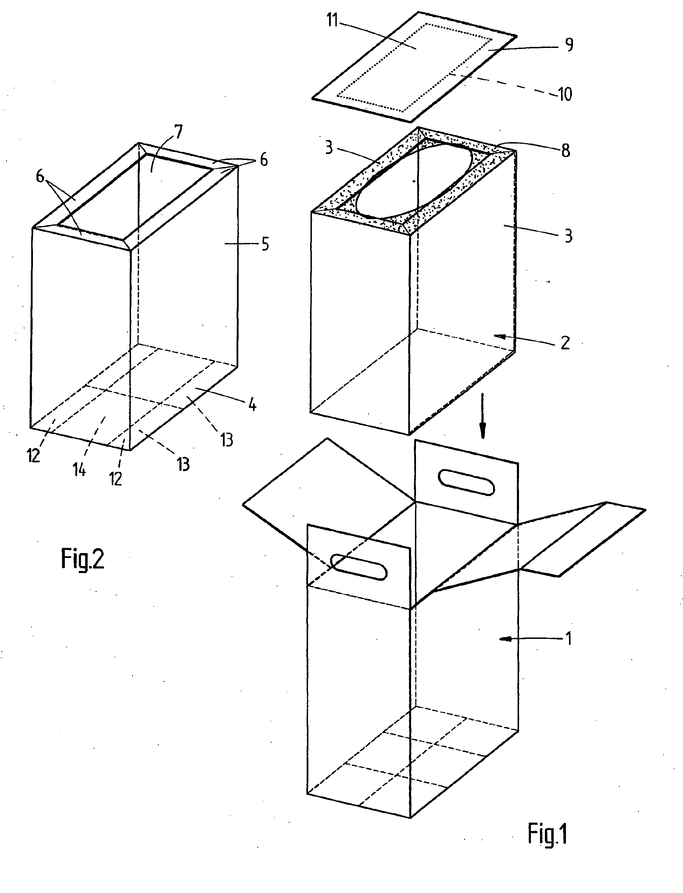

The drawing shows an embodiment of the container for collecting and transporting special waste. Said container comprises a closable outer box 1 made of cardboard, an inner box 2 made of cardboard and a liner of plastic material 3 positioned between the outer box 1 and the inner box 2. The inner box 2 of the container as described herein, which is shown without the liner 3 of plastic material in Fig. 2, consists of a waterproof, solid cardboard and comprises a closed bottom 4, upright side walls 5 and end flaps 6 extending inwardly from the side walls 5, which end flaps bound a filling opening 7. The waterproof, solid cardboard of the inner box 2 and the outer box 1 is in particular a solid, waterproof, laminated cardboard provided with a coating of plastic material. The liner 3 of plastic material is a foil which is provided around the bottom 4, the side walls 5 and the end flaps 6 with a close fit, as is schematically shown in Fig. 1. The foil 3 is in particular a shrink foil, which is wrapped around the inner box 2 upon forming of the container described herein, after which the shrink foil is sealed by welding on the side of the bottom 4, using a single or a multiple weld. Then the inner box 2 and the foil 3 that is wrapped around said inner box are subjected to a heat treatment, as a result of which the foil 3 is pulled over the inner box 2 with a close fit. The extent to which the shrink foil 3 projects on the side of the filling opening 7 has been selected so that the filling opening 7 is left substantially clear after said heat treatment. The shrink foil 3 still abuts closely against the end flaps 6 in that situation. An adhesive 8 has been applied to the shrink foil 3 that is present on the end flaps 6, which adhesive is covered by a removable protective paper 9, which is impregnated with silicones, for example, to prevent adherence. The protective paper 9 may be provided with a perforation 10 (schematically indicated) so as to leave the filling opening 7 clear. It is also possible, of course, to remove the central portion 11 after the protective paper 9 has been applied. The adhesive 8 is preferably a hotmelt, which, in combination with the shrink foil 3 and the plastic-coated cardboard, ensures an excellent, liquid-tight seal.

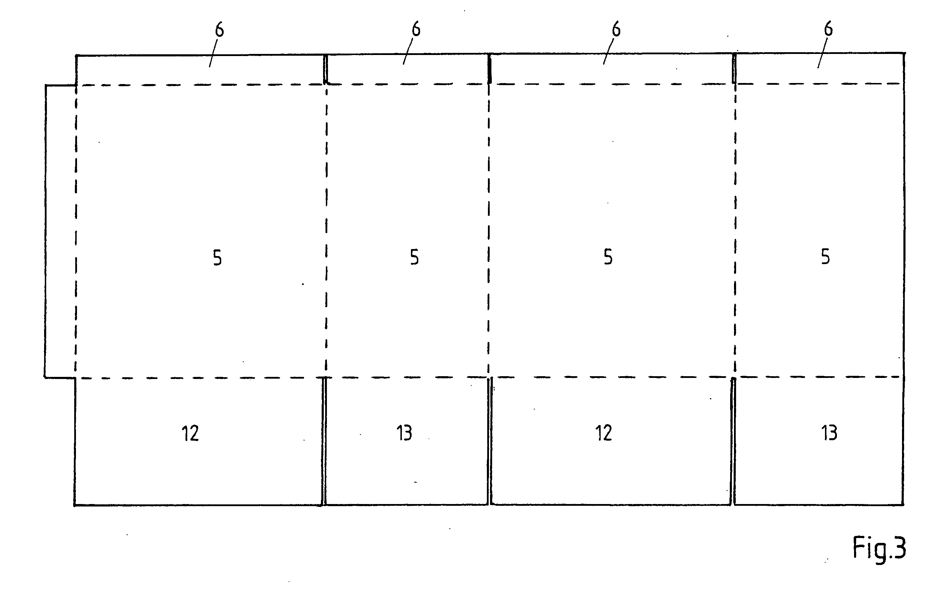

The inner box 2 is formed of the blank that is shown in Fig. 3, with the bottom 4 being formed of the bottom flaps 12 of the long side walls 5 and the bottom flaps 13 of the short side walls 5. The length of the bottom flaps 12 is such that said bottom flaps partially overlap, as is indicated at 14 in Fig. 2. In this way a fully closed bottom 4 is guaranteed, so that sharp objects cannot pierce the shrink foil 3.

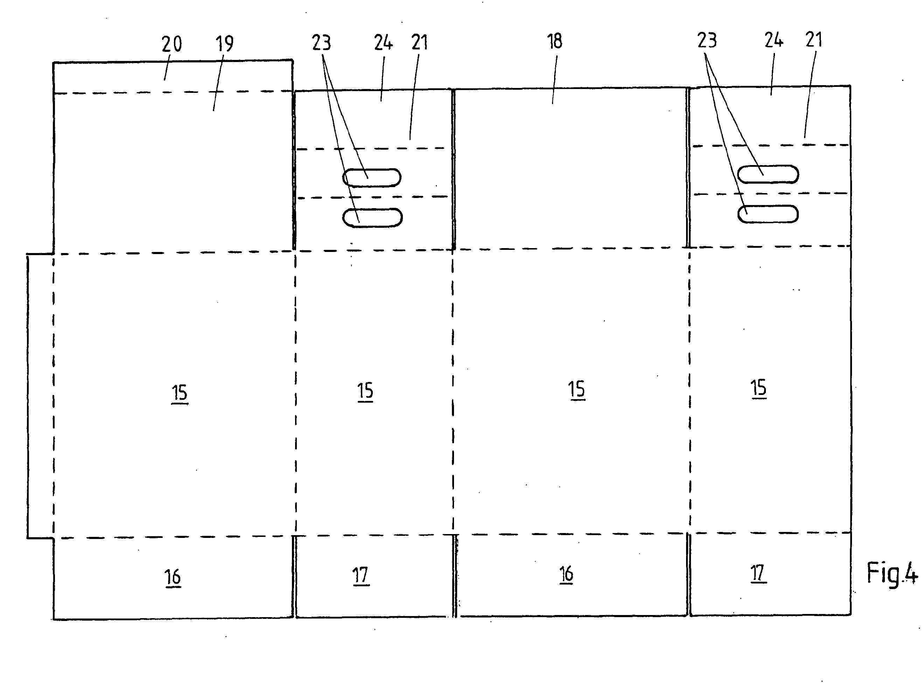

The outer box 1 is formed of a blank of a waterproof, solid cardboard that is shown in Fig. 4. Preferably, an unpierceable, waterproof, solid cardboard is used both for the inner box 2 and for the outer box 1, preferably minimally 750g cardboard. The thickness of the material of the solid cardboard of the inner box and the outer box combined meets the requirements of resistance to piercing in accordance with British Standard BS 7320 (1990).

As is apparent from Figs. 1 and 4, the upright side walls 15 are provided with bottom flaps 16 and 17, respectively, which form the closed bottom of the outer box 1, using sealing tape, in a so-called standard American closure. At the upper side, the long side walls 15 are provided with a closing flap 18 for the inner box 2 and an upper flap 19, which may be used as a cover for the outer box 1 and which covers the closing flap 18 in the closed position. In the illustrated embodiment, the upper flap 19 is provided with an adhesive (not shown), preferably a layer of hotmelt, on the inner side. Said adhesive is covered by a silicone-impregnated protective paper (likewise not shown). If desired, the upper flap 19 may be provided with an extension 20, which is likewise provided with an adhesive covered by a protective paper. Said extension 20 may be attached to the outer side of the outer box 1 in that case. It is possible to provide only the extension 20 of the upper flap 19 with an adhesive.

The two short side walls 15 of the outer box 1 are provided with upper flaps 21 having a folding seam 22. Said upper flaps 21 are each provided with two grip openings 23. The upper flaps 21 are folded about the folding seam 22, so that the grip openings 23 will be positioned opposite each other and the end portions 24 will come to abut against the inner side of the side walls 15.

A unit of absorption material, which absorbs the moisture in the waste, may be present in the container, if desired. This provides additional safety as regards the risk of moisture leaking from the container.

The dimensions of the outer box 1 and the inner box 2 are such that the inner box 2 can easily be placed into the outer box 1 and, with the inner box positioned within the outer box, the closing flap 18 can be adhered to the end flaps in a flat position. The dimensions (1 x w x h) of the inner box 2 are 387 x 287 x 490 mm, for example, and the dimensions of the outer box are 394 x 292 x 495 mm. In use, the waste can be deposited into the inner box 2 of the container via the filling opening 7, with the protective paper 9 protecting the layer of hotmelt 8 that is present on the end flaps 6 of the inner box. When the container is to be closed, the protective paper 9 is removed and the closing flap 18 is affixed or attached to the end flaps 6, thus ensuring a watertight, leak-proof seal. Subsequently, the protective paper present on the inner side of the upper flap 19 is removed, and the upper flap 19 is attached to the closing flap 18. Following this, the container may be stored and be transported to the waste processing plant, in particular in the manner as described in the aforesaid European patent application EP-A-1 153 855.

The invention is not limited to the embodiments as described above, which can be varied in many ways within the scope of the invention as defined in the claims.

## Claims

1. A contained for collecting and transporting special waste, in particular hospital waste, comprising a closable outer box (1) made of cardboard, an insert made of card-board, which is capable of being placed in the outer box (1), and a liner (3) of plastic material, which is positioned between the outer box and the insert, the insert being constructed as an inner box (2) formed of a waterproof, solid cardboard, which comprises a closed bottom (4) and upright side walls (5) having inwardly extending end flaps (6) that bound a filling opening (7), the liner of plastic material being a foil which is provided on the bottom (4), the side walls (5) and the end flaps (6) of the inner box (2) with a close fit, **characterized in that** the foil on the end flaps (6) has an adhesive (8) whereby the fill-ing opening (7) is capable of being closed by means of a closing flap (18) of a waterproof, solid cardboard which is capable of being sealingly attached to the adhesive (8).

2. A container according to claim 1, wherein said foil is a shrink foil.

3. A container according to claim 1 or 2, wherein said adhesive is a hotmelt.

4. A container according to any one of the preceding claims, wherein the outer box (1) is formed of a blank of a waterproof, solid cardboard, comprising two upper flaps, which are connected with opposed side walls, wherein a first upper flap is embodied as the closing flap (18) for the filling opening of the inner box (2) and wherein the second upper flap is embodied as the cover for the outer box (1).

5. A container according to claim 4, wherein the second upper flap is provided with an adhesive, preferably a hotmelt, on the inner side.

6. A container according to claim 4 or 5, wherein the second upper flap is provided with a closing flap which joins the side wall in the closed position of the second upper flap, and which is provided with an adhesive, preferably a hotmelt, on the inner side.

7. A container according to any one of the preceding claims, wherein the inner box (2) is provided with at least two bottom flaps, which are connected to opposed side walls and which at least partially overlap.

8. A container according to any one of the preceding claims, wherein two opposed side walls of the outer box are provided with carrying flaps comprised of side wall flaps that are folded in two, whose ends abut against the inner side of the side walls.

9. A container according to any one of the preceding claims, wherein the inner box and the outer box are made of a solid cardboard that is coated with a plastic on at least one side.

10. A container according to any one of the preceding claims, wherein an amount of absorption material is present in the container.

11. A method for manufacturing a container according to any one of the preceding claims, wherein the inner box (2) is wrapped in a shrink foil, which shrink foil is sealed at the bottom side by welding, wherein said shrink foil is pulled over the bottom, the side walls and the end flaps of the inner box (2) with a close fit, by a heat treatment, wherein the adhesive (8), preferably a hotmelt, is applied to the end flaps (6), which adhesive (8) is covered by a removable protective layer (9).

12. A method according to claim 11, wherein the outer box (1) comprises upper flaps on two opposed side walls is provided, wherein the first upper flap functions as a closing flap for the inner box (2) and wherein the second upper flap, which functions as a closing flap for the outer box, is provided with an adhesive, preferably a layer of hotmelt, which is covered by a removable protective layer.

## Patentansprüche

1. Behälter zum Sammeln und Transportieren von Sondermüll, insbesondere Krankenhausmüll, der eine verschließbare Außenschachtel (1), die aus Kartonpapier hergestellt ist, einen aus Karton hergestellter Einsatz, der in der Außenschachtel (1) anordenbar ist, und eine zwischen der Außenschachtel und dem Einsatz angeordnete Zwischenlage (3) aus Kunststoffmaterial umfasst, wobei der Einsatz als eine aus einem wasserdichten, festen Karton ausgebildete Innenschachtel (2) ausgebildet ist, die einen geschlossenen Boden (4) und senkrechte Seitenwände (5) mit sich einwärts erstreckenden Endklappen (6) aufweist, die eine Einfüllöffnung (7) begrenzen, und wobei die Zwischenlage aus Kunststoffmaterial eine Folie ist, die auf dem Boden (4), den Seitenwände (5) und den Endklappen (6) der Innenschachtel (2) eng anliegend angeordnet ist, **dadurch gekennzeichnet, dass** die Folie auf den Endklappen (6) ein Klebemittel (8) aufweist, wodurch die Einfüllöffnung (7) mittels einer aus einem wasserdichten, festen Karton bestehenden Verschlussklappe (18), die abdichtend an das Klebemittel (8) anlegbar ist, verschließbar ist.

2. Behälter nach Anspruch 1, wobei die Folie eine Schrumpffolie ist.

3. Behälter nach Anspruch 1 oder 2, wobei das Klebemittel ein Heißschmelzkleber ist.

4. Behälter nach einem der vorhergehenden Ansprüche, wobei die Außenschachtel (1) aus einem Zuschnitt aus einem wasserdichten, festen Karton gebildet ist, der zwei obere Klappen umfasst, die mit gegenüberliegenden Seitenwänden verbunden sind, wobei eine erste obere Klappe als Verschlussklappe (18) für die Einfüllöffnung der Innenschachtel (2) ausgebildet ist und wobei die zweite obere Klappe als Abdeckung für die Außenschachtel (1) ausgebildet ist.

5. Behälter nach Anspruch 4, wobei die zweite obere Klappe auf der Innenseite mit einem Klebemittel, vorzugsweise einem Heißschmelzkleber, versehen ist.

6. Behälter nach Anspruch 4 oder 5, wobei die zweite obere Klappe mit einer Verschlussklappe versehen ist, die in der geschlossenen Position der zweiten oberen Klappe an der Seitenwand anliegt und die auf der Innenseite mit einem Klebemittel, vorzugsweise mit einem Heißschmelzkleber, versehen ist.

7. Behälter nach einem der vorhergehenden Ansprüche, wobei die Innenschachtel (2) mit wenigstens zwei Bodenklappen versehen ist, die mit gegenüberliegenden Seitenwänden verbunden sind und die sich zumindest teilweise überdecken.

8. Behälter nach einem der vorhergehenden Ansprüche, wobei zwei gegenüberliegende Seitenwände der Außenschachtel mit Trageklappen versehen sind, die in zwei Klappen gefaltetet sind, deren Enden gegen die Innenseite der Seitenwände anliegen.

9. Behälter nach einem der vorhergehenden Ansprüche, wobei die Innenschachtel und die Außenschachtel aus einem festen Karton hergestellt sind, das mit einem Kunststoff auf wenigstens einer Seite beschichtet ist.

10. Behälter nach einem der vorhergehenden Ansprüche, wobei eine Menge an Absorptionsmaterial in dem Behälter vorhanden ist.

11. Verfahren zur Herstellung eines Behälters gemäß einem der vorhergehenden Ansprüche, wobei die Innenschachtel (2) in eine Schrumpffolie eingewickelt wird, wobei die Schrumpffolie an der Bodenseite durch Verschweißen versiegelt wird, wobei die Schrumpffolie durch eine Wärmebehandlung eng anliegend über den Boden, die Seitenwände und die Endklappen der Innenschachtel (2) gezogen wird, wobei das Klebemittel (8), vorzugsweise ein Heißschmelzkleber, auf die Endklappen (6) aufgebracht wird, wobei das Klebemittel (8) von einer lösbaren Schutzschicht (9) abgedeckt wird.

12. Verfahren nach Anspruch 11, wobei die Außenschachtel (1) an zwei gegenüberliegenden Seitenwänden mit oberen Klappen versehen ist, wobei die erste obere Klappe als eine Verschlussklappe für die Innenschachtel (2) fungiert und wobei die zweite obere Klappe, die als eine Verschlussklappe für die Außenschachtel fungiert, mit einem Klebemittel, vorzugsweise einer Schicht aus Heißschmelzkleber, versehen ist, das von einer lösbaren Schutzschicht abgedeckt ist.

## Revendications

1. Conteneur pour recueillir et transporter des déchets spéciaux, en particulier des déchets hospitaliers, comprenant un caisson extérieur obturable (1) réalisé en carton, un insert réalisé en carton, qui peut être placé dans le caisson extérieur (1), et une doublure (3) en matière plastique, qui est positionnée entre le caisson extérieur et l'insert, l'insert étant construit comme un caisson interne (2) formé en un carton solide étanche à l'eau qui comprend un fond fermé (4) et des parois latérales verticales (5) ayant des rabats d'extrémité s'étendant vers l'intérieur (6) qui délimitent une ouverture de remplissage (7), la garniture en matériau plastique étant une feuille qui est disposée sur le fond (4), des parois latérales (5) et les rabats d'extrémité (6) du caisson interne (2) en ajustage serré, **caractérisé en ce que** la feuille sur les rabats d'extrémité (6) présente une matière adhésive (8), de sorte que l'ouverture de remplissage (7) est fermée au moyen d'un rabat de fermeture (18) en carton solide étanche à l'eau qui peut être fixé de façon hermétique sur la partie adhésive (8) .

2. Conteneur selon la revendication 1, dans lequel ladite feuille est une feuille rétractable.

3. Conteneur selon la revendication 1 ou 2, dans lequel ladite partie adhésive est une substance thermo-fusible.

4. Conteneur selon l'une quelconque des revendications précédentes, dans lequel le caisson extérieur (1) est formé d'une ébauche en carton solide étanche à l'eau, comprenant deux rabats supérieurs qui sont raccordés aux parois latérales opposées, dans lequel un premier rabat supérieur est conçu comme rabat de fermeture (18) pour l'ouverture de remplissage du caisson interne (2) et dans lequel le second rabat supérieur est conçu comme couvercle pour le caisson extérieur (1).

5. Conteneur selon la revendication 4, dans lequel le second rabat supérieur est muni d'une substance adhésive, de préférence une substance thermo-fusible sur le côté intérieur.

6. Conteneur selon la revendication 4 ou 5, dans lequel le second rabat supérieur est muni d'un rabat de fermeture qui relie la paroi latérale dans la position fermée du second rabat supérieur et qui est muni d'une substance adhésive, de préférence une substance thermo-fusible, sur le côté intérieur.

7. Conteneur selon l'une quelconque des revendications précédentes, dans lequel le caisson interne (2) est muni d'au moins deux rabats de fond qui sont raccordés aux parois latérales opposées et qui se chevauchent au moins partiellement.

8. Conteneur selon l'une quelconque des revendications précédentes, dans lequel deux parois latérales opposées du caisson externe sont munies de rabats de support constitués de parois latérales qui sont repliées en deux, dont les extrémités viennent en butée contre le côté interne des parois latérales.

9. Conteneur selon l'une quelconque des revendications précédentes, dans lequel le caisson interne et le caisson externe sont réalisés à partir d'un carton solide qui est revêtu d'un plastique sur au moins un côté.

10. Conteneur selon l'une quelconque des revendications précédentes, dans lequel une quantité de matériau d'absorption est présente dans le conteneur.

11. Procédé pour la fabrication d'un conteneur selon l'une quelconque des revendications précédentes, dans lequel le caisson interne (2) est enroulé dans une feuille rétractable, laquelle feuille rétractable est raccordée de façon étanche par soudage sur le côté inférieur, dans lequel ladite feuille rétractable est rabattue sur le fond, les parois latérales et les rabats d'extrémité du caisson interne (2) sont ajustés serrés par un traitement thermique, dans lequel la matière adhésive (8), de préférence une substance thermo-fusible, est appliquée sur les rabats d'extrémité (6), laquelle matière adhésive (8) est revêtue d'une couche protectrice amovible (9).

12. Procédé selon la revendication 11, dans lequel le caisson externe (1) comprend des rabats supérieurs sur les deux parois latérales opposées, dans lequel le premier rabat supérieur sert de rabat de fermeture pour le caisson interne (2) et dans lequel le second rabat supérieur qui sert de rabat de fermeture pour le caisson extérieur est muni d'une substance adhésive, de préférence d'une couche thermo-fusible qui est revêtue d'une couche protectrice amovible.
